(19) Europäisches Patentamt
European Patent Office
Office européen des brevets

(11) Numéro de publication : **0 463 970 A1**

(12) # DEMANDE DE BREVET EUROPEEN

(21) Numéro de dépôt : **91401741.3**

(22) Date de dépôt : **27.06.91**

(51) Int. Cl.[5] : **C07D 498/04,** C07D 213/76, A61K 31/44, // (C07D498/04, 263:00, 221:00)

(30) Priorité : **29.06.90 FR 9008202**

(43) Date de publication de la demande : **02.01.92 Bulletin 92/01**

(84) Etats contractants désignés : **AT BE CH DE DK ES FR GB GR IT LI LU NL SE**

(71) Demandeur : **ADIR ET COMPAGNIE**
**1 rue Carle Hébert**
**F-92415 Courbevoie Cédex (FR)**

(72) Inventeur : **Guillaumet, Gérald**
**2 rue Auguste Renoir**
**F-45100 Orleans (FR)**
Inventeur : **Flouzat, Christine**
**9 rue Barrière de Jaude**
**F-63000 Clermont-Ferrand (FR)**
Inventeur : **Caignard, Daniel-Henri**
**69 bis rue Brancion**
**F-75015 Paris (FR)**
Inventeur : **Renard, Pierre**
**50 avenue de Villeneuve l'Etang**
**F-78000 Versailles (FR)**
Inventeur : **Devissaguet, Michelle**
**14 Bld d'Inkerman**
**F-92200 Neuilly Sur Seine (FR)**
Inventeur : **Guardiola, Béatrice**
**6 rue Edouard Nortier**
**F-92200 Neuilly Sur Seine (FR)**

(54) **Nouveaux dérivés d'oxazolo pyridines, leur procédé de préparation et les compositions pharmaceutiques qui les contiennent.**

(57) Dérivés de formule générale (I) :

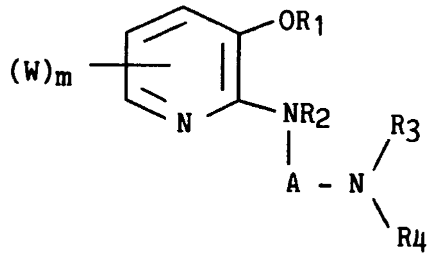

(I)

dans laquelle :

$R_1$ et $R_2$ représentent chacun un atome d'hydrogène ou forment avec l'azote et l'oxygène qui les portent, un chaînon - O - CO - N,

W représente un atome d'halogène ou un groupement alkyle ou alkoxy inférieur éventuellement substitué par un ou plusieurs atomes d'halogène tel que trifluorométhyle, et m étant compris entre 0 et 3,

A est un radical alkyle linéaire ou ramifié comprenant de 1 à 6 atomes de carbone,

$R_3$ et $R_4$ définis dans la description.

Médicament.

EP 0 463 970 A1

La présente invention concerne de nouveaux dérivés des oxazolo - [4,5b] pyridines, leur procédé de préparation et les compositions pharmaceutiques qui les contiennent.

On connaît déjà les propriétés à la fois analgésiques et anti-inflammatoires des 2-phényl [3H] oxazolo - [5,4], [4,5] pyridines (brevets US 4 038 396, Fr 2 328 471, Fr 2 319 354, GB 1 421 619).

Toutefois, ces produits possèdent un profil essentiellement anti-inflammatoire comme le confirment les indications thérapeutiques mentionnées dans les brevets cités ci-dessus ou bien présentent l'inconvénient de ne pas dissocier les deux types d'activités : analgésique d'une part, anti-pyrétique, anti-inflammatoire d'autre part.

La demanderesse a maintenant découvert de nouveaux composés de bon niveau analgésique mais possédant la caractéristique particulièrement avantageuse d'être totalement dépourvus d'activité anti-inflammatoire : les composés de la présente invention sont en effet doués d'une activité analgésique pure de haut niveau. Or, la plupart des substances analgésiques non morphiniques connues à ce jour possèdent également une activité anti-inflammatoire (exemples : salicylés, pyrazolés...) et ils interviennent par conséquent sur les processus intervenant dans l'inflammation. Ceux-ci impliquent de très nombreux médiateurs chimiques (prostaglandines, thromboxane A2...) ; il s'ensuit donc de multiples effets secondaires dont les plus connus sont : attaque de la muqueuse gastrique avec possibilité d'ulcères, inhibition de l'agrégation plaquettaire avec troubles de la coagulation. Outre les dérangements qu'ils occasionnent, ces effets parallèles interdisent l'usage de ces produits chez de nombreux sujets qui y sont particulièrement sensibles. En étant dépourvus de toute activité anti-inflammatoire, les composés de la présente invention n'interviennent donc pas sur les médiateurs de l'inflammation et sont donc dépourvus des effets secondaires mentionnés précédemment. Cette caractéristique, jointe à leur absence totale de toxicité et à leur haut niveau d'activité rend les composés de la présente invention utilisables en tant qu'analgésique d'une façon beaucoup plus sûre et sans les restrictions d'usage habituellement connues pour la grande majorité de ces produits. En outre, certains produits de l'invention ont révélé une affinité pour les récepteurs muscariniques, ce qui les distingue totalement de l'art antérieur.

Plus spécifiquement l'invention concerne des dérivés de formule générale (I) :

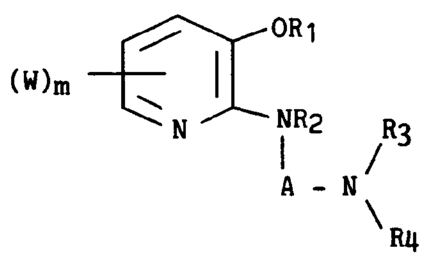

(I)

dans laquelle :

$R_1$ et $R_2$ représentent chacun un atome d'hydrogène ou forment avec l'azote et l'oxygène qui les portent, un chaînon - O - CO - N,

W représente un atome d'halogène ou un groupement alkyle ou alkoxy inférieur éventuellement substitué par un ou plusieurs atomes d'halogène tel que trifluorométhyle, et m étant compris entre 0 et 3,

A est un radical alkyle linéaire ou ramifié comprenant de 1 à 6 atomes de carbone,

$R_3$ et $R_4$ identiques ou différents représentent :

– un atome d'hydrogène,
– un groupement alkyle inférieur linéaire ou ramifié,
– un groupement alkényle inférieur linéaire ou ramifié,
– un groupement cycloalkyle de 6 à 10 atomes de carbone,
– un groupement aryle ou alkyle inférieur aryle ou arylalkyl inférieur,

chacun de ces groupements étant substitué ou non par un ou plusieurs atomes d'halogène ou groupements trifluorométhyle, hydroxy ou alkoxy inférieurs,

ou bien :

$R_3$ et $R_4$ constituent avec l'atome d'azote auquel ils sont liés un système hétérocyclique azoté mono ou bicyclique, saturé ou non comprenant un maximum de 12 atomes - non compris les atomes d'hydrogène - parmi lesquels on peut compter un à trois hétéroatomes choisis parmi azote, oxygène ou soufre substitué ou non par un groupement alkyle inférieur ou phényle ou phénylalkyle inférieur ou diphénylalkyle inférieur, les groupements phényle, phénylalkyle inférieur ou diphénylalkyle inférieur pouvant être substitués par un ou plusieurs atomes d'halogène ou groupements hydroxy, alkyle inférieurs, alkoxy inférieurs ou trifluorométhyle, à la condition que $R_3$ et $R_4$ ne constituent pas avec l'atome d'azote auquel ils sont liés un système aryl-1 pipérazinique

ou hétéroaryl-1 pipérazinique,
étant entendu que par radical alkyle inférieur, alkényle inférieur, ou alkyloxy inférieur, on entend un groupement linéaire ou ramifié comprenant de 1 à 6 atomes de carbone et que par groupements aryle ou hétéroaryle, on entend des groupements mono ou bicycliques insaturés comprenant de 5 à 12 atomes - non compris les atomes d'hydrogène - intégrant ou non dans leur squelette carboné un, deux ou trois hétéroatomes choisis parmi azote, oxygène ou soufre,
leurs isomères, épimères, diastéréoisomères,
ainsi que leurs sels d'addition à un acide pharmaceutiquement acceptable et lorsque $R_1$ et $R_2$ représentent chacun un atome d'hydrogène, leurs sels d'addition à une base pharmaceutiquement acceptable.

Parmi les acides que l'on peut ajouter aux composés de formule (I) pour former un sel d'addition, on peut citer à titre d'exemple, les acides chlorhydrique, sulfurique, phosphorique, tartrique, malique, maléique, fumarique, oxalique, méthane sulfonique, éthane sulfonique, camphorique, citrique...

Parmi les bases que l'on peut ajouter aux composés de formule (I) pour lesquels $R_1$ et $R_2$ représentent chacun un atome d'hydrogène on peut citer à titre d'exemple, les hydroxydes de métal alcalin, les sels de métal alcalin...

L'invention s'étend également au procédé d'obtention de composés de formule (I), caractérisé en ce que l'on fait réagir un dérivé de formule (II) :

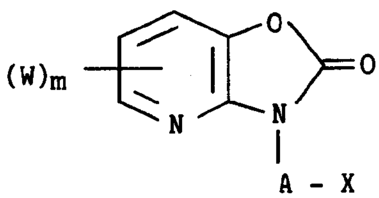

(II)

dans lequel W, m et A ont la même signification que dans la formule (I) et X représente un atome d'halogène, préférentiellement sous atmosphère inerte avec un dérivé de formule (III), préférentiellement en excès :

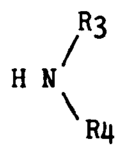

(III)

dans laquelle $R_3$ et $R_4$ ont la même signification que dans la formule (I),
en milieu organique en présence d'un agent basique et à température comprise entre la température ambiante et la température du reflux du solvant choisi pour conduire après refroidissement, extraction et éventuelle purification à un dérivé de formule (I/A) :

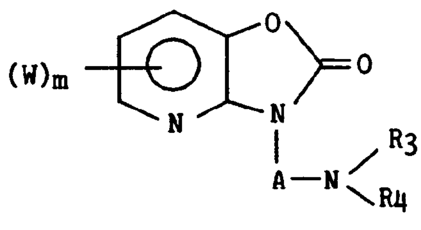

(I/A)

cas particulier des dérivés de formule (I) pour lesquels $R_1$ forme avec $R_2$ un groupement C = O,
que l'on peut, si on le désire, séparer le cas échéant, en ses isomères puis salifier par un acide pharmaceutiquement acceptable,
dérivé de formule (I/A) que l'on peut traiter, si on le désire, par un agent alcalin en solution aqueuse, à une température comprise entre la température ambiante et la température d'ébullition du milieu réactionnel pour conduire, après éventuelle acidification et/ou neutralisation du milieu réactionnel à un dérivé de formule (I/B) :

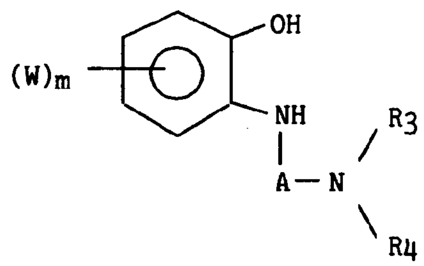

(I/B)

cas particulier des dérivés de formule (I) pour lesquels $R_1$ et $R_2$ représentent chacun un atome d'hydrogène et W, m, A, $R_3$ et $R_4$ ont la même signification que précédemment que l'on purifie, si nécessaire par une technique choisie parmi cristallisation ou chromatographie et que l'on salifie, si on le désire par un acide ou une base pharmaceutiquement acceptables.

Les dérivés de formule (II) pourront être obtenus par mise en réaction d'un dérivé de formule (IV) :

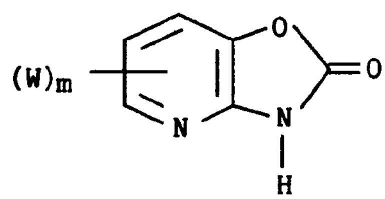

(IV)

dans laquelle W et m ont la même signification que dans la formule (I),
avec un hydroxyde de métal alcalin en milieu aqueux ou un alcoolate de métal alcalin en milieu organique, pour conduire à un dérivé de formule (V) :

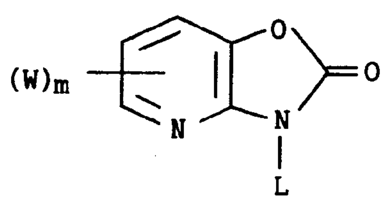

(V)

dans laquelle W et m ont la même signification que précédemment, et L représente un métal alcalin,
que l'on condense avec un dérivé de formule (VI) :

X-A-X′ (VI)

dans laquelle A a la même signification que précédemment, et X et X′ identiques ou différents représentent chacun un atome d'halogène,
préférentiellement sous atmosphère inerte, en milieu organique à une température comprise entre la température ambiante et le reflux du solvant choisi pour conduire après éventuelle extraction et purification par chromatographie et/ou cristallisation au dérivé de formule (II).

Les dérivés de formule (I/A) pourront également être obtenus par condensation d'un dérivé de formule (V) telle qu'indiquée précédemment avec un dérivé de formule (VII) :

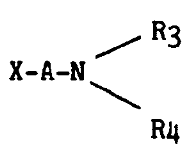

(VII)

dans laquelle X, A et $R_3$, $R_4$ ont la même signification que précédemment,
en milieu organique à température comprise entre la température ambiante et la température du reflux du solvant choisi pour conduire, après éventuellement refroidissement, extraction, purification à un dérivé de formule (I/A) telle que définie précédemment dont on sépare le cas échéant les isomères et que l'on salifie, si on le désire par un acide pharmaceutiquement acceptable.

Les dérivés de formule (VII) seront avantageusement obtenus par condensation d'un dérivé de formule (VI) telle que précédemment définie sur une amine de formule (III) telle que précédemment définie préférentiellement sous atmosphère inerte, en milieu organique à une température comprise entre la température ambiante et le reflux du solvant choisi pour conduire après éventuelle extraction et purification à un dérivé de formule (VII).

Un cas particulier des dérivés de la présente invention est constitué par les dérivés de formule (I) pour lesquels A est un chaînon méthylène $-CH_2-$.

Ces dérivés seront avantageusement obtenus en une seule étape par dissolution, en milieu d'alcool aliphatique inférieur, d'un dérivé de formule (IV), d'une amine de formule (III) en léger excès, et d'un excès de formol, chauffage de la solution ainsi obtenue à une température comprise entre la température ambiante et la température d'ébullition de la solution,
pour conduire, après éventuel refroidissement, repos d'une à deux heures, filtration, et éventuelle chromatographie sur colonne de silice, à un dérivé de formule (I/A1) :

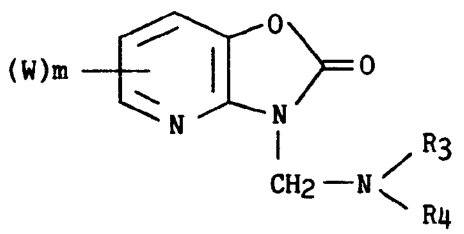

**(I/A1)**

cas particulier des dérivés de formule (I/A) pour lequel A est un chaînon méthylène $-CH_2-$, et dans laquelle W et m ont la même signification que dans la formule (I), que l'on peut salifier, si on le désire par un acide pharmaceutiquement acceptable et transformer si on le désire en dérivé (I/B1) :

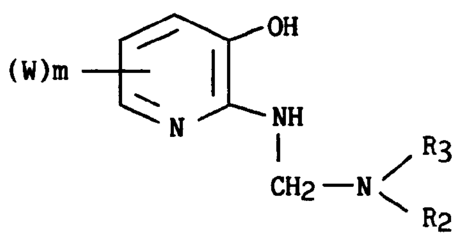

**(I/B1)**

cas particulier des dérivés de formule (I/B) pour lequel A est un chaînon méthylène $CH_2$, W et m ont la même signification que dans la formule (I), par traitement par un agent alcalin en solution aqueuse d'un dérivé de formule (I/A1) comme indiqué pour la transformation des dérivés de formule (I/A) en dérivé de formule (I/B).

Les composés de formule (I) possèdent des propriétés pharmacologiques intéressantes.

En particulier, ces dérivés ont révélé une activité analgésique intéressante.

L'étude pharmacologique des dérivés de l'invention a montré qu'ils étaient peu toxiques, doués d'une activité analgésique pure de haut niveau, dépourvue de composante anti-inflammatoire et donc dépourvue d'inconvénients inhérents à la plupart des composés présentant cette activité (action ulcérigène sur les muqueuses, perturbation de la coagulation...). Ce spectre d'activité rend donc les composés de la présente invention particulièrement intéressants dans un certain nombre d'indications telles que algies rhumatismales telles que entorses, fractures, luxations, douleurs post-traumatiques, douleurs post-opératoires, douleurs dentaires, douleurs neurologiques telles que névralgies faciales, douleurs viscérales telles que coliques néphrétiques, dysménorrhées, chirurgie proctologie, pancréatite, algies diverses, céphalées, douleurs des cancéreux...

En outre, les composés de l'invention ont révélé une bonne affinité pour les récepteurs M1. Ce qui signifie qu'ils peuvent être utilisés avec profit dans les troubles de l'insuffisance circulatoire cérébrale, les multiples désordres résultant du vieillissement normal ou pathologique, la perte de mémoire, et la maladie d'Alzheimer.

La présente invention a également pour objet les compositions pharmaceutiques contenant les produits de formule (I) ou un de leurs sels d'addition à un acide pharmaceutiquement acceptable, seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, rectale, perlinguale, oculaire ou respiratoire, et notamment les préparations injectables, les aérosols, les gouttes oculaires ou nasales, les comprimés simples

ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la voie d'administration, la nature de l'indication thérapeutique et des traitements éventuellement associés et s'échelonne entre 1 centigramme et 4 grammes par 24 heures.

Les exemples suivants illustrent l'invention et ne la limitent en aucune façon.

Les spectres de résonance magnétique nucléaire du $^{1}H$ ont été enregistrés en utilisant le TMS comme référence interne. Les spectres infrarouges ont été enregistrés à partir d'une pastille de KBr renfermant 1% environ du produit à analyser.

Les produits obtenus selon les protocoles opératoires décrits dans la rubrique "Préparations" ne font pas partie de l'invention, ils constituent néanmoins des intermédiaires de synthèse utiles pour la préparation des composés de l'invention.

**PREPARATIONS :**

**PREPARATION 1 : [3H] OXAZOLO [4,5b] PYRIDIN-2-ONE ***

Dans un tricol, verser 5,5 g (0,05 M) de 2-amino 3-hydroxy pyridine et mettre le système sous argon. Additionner 100 ml de tétrahydrofuranne anhydre (THF). Verser ensuite 12,15 g (0,075 M) de 1,1 - carbonyldiimidazole. Chauffer à reflux pendant 5 heures (sous argon). Evaporer ensuite le THF. Reprendre le résidu par du dichlorométhane. Effectuer des lavages de la phase organique avec une solution de NaOH (5%) (6 x 150 ml), le produit cyclisé passe dans la phase aqueuse et précipité à pH voisin de 5 (par addition d'une solution 2N d'acide chlorhydrique). Filtrer et conserver au dessicateur.
Rendement : 77 %
Point de fusion : 212 - 214 °C

**PREPARATION 2 : 5-METHYL [3H] OXAZOLO [4,5b] PYRIDIN-2-ONE**

**STADE A : 2-NITRO 3-HYDROXY 6-METHYL PYRIDINE**

Ajouter 5,45 g (50 mM) de 5-hydroxy 2-méthyl pyridine dans 20 ml d'acide sulfurique concentré, en refroidissant dans un bain de glace. Maintenir la température à +6°C et ajouter 2,35 ml d'acide nitrique fumant, sous agitation. Laisser une nuit à température ambiante. Ajouter 100 g de glace sous agitation. Filtrer et rincer à l'eau, sécher.

**STADE B : 2-AMINO 3-HYDROXY 6-METHYL PYRIDINE**

Placer sous pression d'hydrogène 3,5 g de 2-nitro 3-hydroxy 6-méthyl pyridine dans 50 ml de méthanol en présence d'un gramme de charbon palladié. Agiter, filtrer. Evaporer le méthanol.

**STADE C : 5-METHYL [3H] OXAZOLO [4,5b] PYRIDIN-2-ONE**

Dans un ballon tricol verser 1,24 g (10 mM) de 2-amino 3-hydroxy 6-méthyl pyridine. Placer sous argon. Additionner 20 ml de tétrahydrofuranne anhydre puis 2,43 g (15 mM) de 1,1-carbonyl diimidazole. Chauffer 6 heures à reflux. Evaporer le milieu réactionnel. Laver les cristaux obtenus à l'eau, filtrer et redissoudre dans du méthanol chaud. Filtrer et réévaporer.
Rendement : 75 %
Point de fusion : 243 °C
Caractéristiques spectrales :
RMN $^{1}H$ Solvant $CDCl_3$ : $\delta$ ppm
$\delta$ : 12,3 1H, massif, NH
$\delta$ : 7,5 1H ; doublet ; $H_7$ ; J = 8 Hz
$\delta$ : 6,9 1H ; doublet ; $H_6$ ; J = 8 Hz
$\delta$ : 2,4 3H ; singulet ; $CH_3$
Infrarouge : 1750 $cm^{-1}$, $\nu$ (C=O)
1610 $cm^{-1}$, $\nu$ (C=C)

* Voir nomenclature en annexe page 24

**PREPARATION 3 : 3-(2-BROMO ETHYL)[3H] OXAZOLO [4,5b] PYRIDIN-3-ONE**

**STADE A : DERIVE SODE DE L'OXAZOLO [4,5b] PYRIDIN-2-ONE**

Dissoudre 6 g (44,11 mM) de [3H] oxazolo [4,5b] pyridin-2-one dans une quantité suffisante de tétrahydrofuranne puis additionner cette solution à une solution éthanolique d'éthylate de sodium obtenue à partir d'un gramme (43,50 mM) de sodium dans environ 150 ml d'éthanol. Evaporer sous vide et reprendre le résidu par une quantité suffisante de diméthylformamide pour le dissoudre.

**STADE B : 3-(2-BROMO ETHYL)[3H] OZAZOLO [4,5b] PYRIDIN-2-ONE**

Dans un ballon sous argon, surmonté d'un réfrigérant, mettre 7,6 ml (88,22 mM) de 1,2- dibromo éthane en solution dans environ 50 ml de diméthylformamide puis additionner lentement, sous agitation, la solution obtenue à l'étape précédente. Porter à 100 °C pendant 2 heures.

Après refroidissement, évaporer sous vide le diméthylformamide puis reprendre le résidu par de l'eau et extraire au chlorure de méthylène. Après séchage sur MgSO4, évaporer le chlorure de methylène et purifier le résidu sur colonne de silice flash (230-240 Mesh) dans le chlorure de méthylène. On obtient après évaporation 5,2 g d'une poudre blanche.

Rendement : 50 %

**PREPARATION 4 : 3-(3-BROMO PROPYL)[3H] OXAZOLO [4,5b] PYRIDIN-2-ONE**

En procédant comme dans la préparation 3, mais en remplaçant au stade B le 1,2-dibromo éthane par le 1,3-dibromo propane, on obtient le produit du titre.

**PREPARATION 5 : 3-(4-BROMO n BUTYL)[3H] OXAZOLO [4,5b] PYRIDIN-2-ONE**

En procédant comme dans la préparation 3, mais en remplaçant au stade B le 1,2-dibromo éthane par le 1,4-dibromo n butane, on obtient le produit du titre.

Rendement : 50 %

Point de fusion : 46 °C

Caractéristiques spectrales :

RMN $^1H$ Solvant $CDCl_3$ : δ ppm

δ : 1,92-2,09 ppm, multiplet ; 4H ; $-CH_2-\underline{CH_2}-\underline{CH_2}-CH_2-Br$

δ : 3,47 ppm, triplet ; 2H ; $\underline{CH_2}-Br$

δ : 3,99 ppm, triplet ; 2H

δ : 7,06 ppm, doublet dédoublé ; 1H ; $H_6$ ; $J_{H6H7}$ = 8,3 Hz ; $J_{H6H5}$ = 5,3 Hz

δ : 7,40 ppm, doublet dédoublé ; 1H ; $H_7$ ; $J_{H7H6}$ = 8,3 Hz ; $J_{H7H5}$ = 1 Hz

δ : 8,11 ppm, doublet dédoublé ; 1H ; $H_5$ ; $J_{H5H6}$ = 5,3 Hz ; $J_{H5H7}$ = 1 Hz

**PREPARATION 6 : 5-METHYL 3-(2-BROMO ETHYL)[3H] OXAZOLO [4,5b] PYRIDIN-2-ONE**

En procédant comme dans la préparation 3, mais en remplaçant la [3H] oxazolo [4,5b] pyridin-2-one par la 5-méthyl [3H] oxazolo [4,5b] pyridin-2-one, on obtient le produit du titre.

**EXEMPLE 1 : 3-(2-PIPERIDINO ETHYL)[3H] OXAZOLO [4,5-b] PYRIDIN-2-ONE**

Dans un ballon placé sous argon et surmonté d'un réfrigérant, on additionne 0,01 mole de 3-(2-bromo éthyl)[3H] oxazolo [4,5-b] pyridin-2-one en solution dans l'acétonitrile, 0,15 mole de pipéridine et 0,015 mole de diisopropyléthylamine. Porter à 80 °C pendant 12 heures. Refroidir, évaporer l'acétonitrile sous vide et reprendre le résidu par de l'eau. Vérifier l'alcalinité du milieu et extraire au dichlorométhane. Sécher sur sulfate

de magnésium, évaporer et recristalliser le résidu.
Rendement : 97 %
Point de fusion : 84 °C
Caractéristiques spectrales :
Infrarouge : 3100-2700 $cm^{-1}$ ν (CH)
1760 $cm^{-1}$ ν (C=O)
1590 $cm^{-1}$ ν (C=C) conjugués
Résonance Magnétique Nucléaire :
RMN $^1H$ Solvant $CDCl_3$ : δ ppm
δ : 1,34-1,56, 6H ; multiplet ; pipéridine (β et ϒ de l'azote)
δ : 2,42-2,55, 4H ; multiplet ; pipéridine (α de l'azote)
δ : 2,76, 2H ; triplet ; $CH_2$-$\underline{CH_2}$, pipéridine ; J = 4,1 Hz
δ : 4,07, 2H ; triplet ; $\underline{CH_2}$,-$CH_2$ ; pipéridine ; J = 6,1 Hz
δ : 7,03 ppm : 1H ; doublet dédoublé ; aromatique ; $H_6$
δ : 7,38 ppm : 1H ; doublet dédoublé ; aromatique ; $H_7$
δ : 8,09 ppm : 1H ; doublet dédoublé ; aromatique ; $H_5$

**EXEMPLE 2 : 3-[2-(4-METHYL PIPERAZIN-1-YL)ETHYL][3H] OXAZOLO [4,5-b] PYRIDIN-2-ONE**

En procédant comme dans l'exemple 1, mais en remplaçant la pipéridine par la 1-méthyl pipérazine, on obtient le produit du titre.
Rendement : 90 %
Point de fusion : 85 °C
Caractéristiques spectrales :
Infrarouge : 3100-2700 $cm^{-1}$ ν (CH)
1760 $cm^{-1}$ ν (C=O)
1590 $cm^{-1}$ ν (C=C) conjugués
Résonance Magnétique Nucléaire :
RMN $^1H$ Solvant $CDCl_3$ : δ ppm
δ : 2,23, singulet ; 3H ; $\underline{CH_3}$
δ : 2,25-2,70, multiplet ; 8H ; pipérazinique
δ : 2,79, 2H ; triplet ; $CH_2$-$\underline{CH_2}$ pipérazine
δ : 4,05, 2H ; triplet ; $\underline{CH_2}$-$CH_2$ pipérazine
δ : 7,04 : 1H ; doublet dédoublé ; aromatique ; $H_6$
δ : 7,39 : 1H ; doublet dédoublé ; aromatique ; $H_7$
δ : 8,09 : 1H ; doublet dédoublé ; aromatique ; $H_5$

**EXEMPLE 3 : 3-[2-(1-PYRROLIDINYL)ETHYL][3H] OXAZOLO [4,5-b] PYRIDIN-2-ONE (OXALATE)**

En procédant comme dans l'exemple 1, mais en remplaçant la pipéridine par la pyrrolidine, on obtient le produit du titre sous forme de base. Dissoudre le produit dans l'éthanol et ajouter 0,01 mole d'acide oxalique. Essorer.
Point de fusion : 180°C
Caracteristiques spectrales :
Infrarouge : 3100-2700 $cm^{-1}$ ν (CH)
1760 $cm^{-1}$ ν (C=O)
1590 $cm^{-1}$ ν (C=C)
Résonance magnétique nucléaire :
RMN $^1H$ Solvant $CDCl_3$ : δ ppm
δ : 7,04 : 1H ; doublet dedoublé ; aromatique ; $H_6$
δ : 7,39 : 1H ; doublet dédoublé ; aromatique ; $H_7$
δ : 8,09 : 1H ; doublet dédoublé ; aromatique ; $H_5$

**EXEMPLE 4 : 3-(2-MORPHOLINO ETHYL)[3H] OXAZOLO [4,5-b] PYRIDIN-2-ONE**

En procédant comme dans l'exemple 1, mais en remplaçant la pipéridine par la morpholine, on obtient le produit du titre.
Rendement : 98 %

Point de fusion : 83 °C
Caractéristiques spectrales :
Infrarouge : 3100-2700 $cm^{-1}$ ν (CH)
1760 $cm^{-1}$ ν (C=O)
1590 $cm^{-1}$ ν (C=C)
Résonance magnétique nucléaire :
RMN $^1H$ Solvant $CDCl_3$ : δ ppm
δ : 2,49-2,53 : 4H ; multiplet ; 2 $CH_2$ en α de l'azote : morpholine
δ : 2,68 : 2H ; $CH_2$-$CH_2$ - morpholine ; J = 6,3 Hz
δ : 3,54-3,58 : 4H ; multiplet ; 2$CH_2$ en α de l'oxygène ; morpholine
δ : 4,04 : 2H ; triplet ; $CH_2$-$CH_2$ - morpholine ; J = 6,3 Hz
δ : 7,03 : 1H ; doublet dédoublé ; aromatique ; $H_6$
δ : 7,38 : 1H ; doublet dédoublé ; aromatique ; $H_7$
δ : 8,08 : 1H ; doublet dédoublé ; aromatique ; $H_5$

**EXEMPLE 5 : 3-(2-AMINO ETHYL)[3H] OXAZOLO [4,5-b] PYRIDIN-2-ONE (CHLORHYDRATE)**

Dans un ballon placé sous argon et surmonté d'un réfrigérant, on additionne 0,013 mole d'hexaméthylène tetramine préalablement dissous dans 20 $cm^3$ de chloroforme et 0,01 mole de 3-(2-bromo éthyl)[3H] oxazolo [4,5-b] pyridin-2-one préalablement dissous dans 15 $cm^3$ de chloroforme. Chauffer à reflux pendant une semaine. Essorer, sécher. Introduire le précipité dans une fiole à col rodé de 250 $cm^3$ munie d'un réfrigérant à reflux, ajouter 50 $cm^3$ d'alcool absolu et 10 $cm^3$ d'acide chlorhydrique concentré. Chauffer deux heures à reflux sous agitation magnétique. Evaporer le solvant au bain-marie sous vide et recristalliser dans l'alcool à 95 °C.
Rendement : 70 %
Caractéristiques spectrales :
Infrarouge : 3200-2400 $cm^{-1}$ ν (NH) et ν (CH)
Résonance Magnétique Nucléaire :
RMN $^1H$ Solvant $CDCl_3$ : δ ppm
δ : 7,05 ppm : 1H ; aromatique ; $H_6$
δ : 7,38 ppm : 1H ; aromatique ; $H_7$
δ : 8,09 ppm : 1H ; aromatique ; $H_5$

**EXEMPLE 6 : 3-[2-(N METHYL N BENZYL) AMINO ETHYL][3H] OXAZOLO[4,5-b] PYRIDIN-2-ONE**

En procédant comme dans l'exemple 1, mais en remplaçant la pipéridine par la (N méthyl N benzyl) amine, on obtient le produit du titre.
Rendement : 75 %
Caractéristiques spectrales :
Infrarouge : 3100-2700 $cm^{-1}$ ν (CH)
1760 $cm^{-1}$ ν
Résonance Magnétique Nucléaire :
RMN $^1H$ Solvant $CDCl_3$ : δ ppm
δ : 2,20 ppm, singulet : 3H ; $CH_3$
δ ; 7,03 ppm : 1H ; $H_6$
δ : 7,38 ppm : 1H ; $H_7$
δ : 8,09 ppm : 1H ; $H_5$
δ ; 7,27 ppm : 5H ; aromatique ($C_6H_5$-$CH_2$)

**EXEMPLE 7 : 3-(2-METHYLAMINO ETHYL)[3H]OXAZOLO [4,5-b] PYRIDIN-2-ONE**

Dans une fiole à col rodé de 1000 $cm^3$, dissoudre 0,02 mole de 3-[2-(N méthyl N benzyl amino) éthyl][3H] oxazolo [4,5-b] pyridin-2-one dans 250 $cm^3$ de méthanol. Introduire 0,2 g de charbon palladié et agiter sous atmosphère d'hydrogène à température et pression ordinaires. Après absorption de la quantité théorique d'hydrogène, filtrer le milieu réactionnel. Concentrer le filtrat au bain-marie sous vide et acidifier par un courant d'acide chlorhydrique gazeux. Essorer le précipité obtenu, sécher et recristalliser.
Rendement : 75 %
Caractéristiques spectrales :
Infrarouge : 3100-2600 $cm^{-1}$ ν (NH) ou ν (CH)

2440 $cm^{-1}$ ν (NH)
1750 $cm^{-1}$ ν CO (OCON)
1610 $cm^{-1}$ ν (C=C) aromatique

Résonance Magnétique Nucléaire :
RMN $^1H$ Solvant $CDCl_3$ : δ ppm
δ : 7,05 ppm, doublet déboublé : 1H ; $H_6$
δ : 7,38 ppm, doublet dédoublé : 1H ; $H_7$
δ : 8,09 ppm, doublet dédoublé : 1H ; $H_5$

**EXEMPLE 8 : 3-(2-ISOPROPYLAMINO ETHYL)[3H]OXAZOLO [4,5-b]PYRIDIN-2-ONE (BROMHYDRATE)**

Dans un ballon à col rodé de 100 $cm^3$ muni d'un réfrigérant à reflux, introduire 0,1 mole d'isopropylamine et 0,01 mole de 3-(2-bromo éthyl) [3H] oxazolo [4,5-b] pyridin-2-one préalablement dissous dans 40 $cm^3$ d'acétonitrile. Chauffer 15 heures à reflux. Après refroidissement, essorer, sécher et recristalliser le précipité obtenu.

Rendement : 92 %

Caractéristiques spectrales :

Infrarouge : 3100-2650 $cm^{-1}$ ν (NH) et ν (CH)
2450 $cm^{-1}$ ν (NH)
1745 $cm^{-1}$ ν CO

**EXEMPLE 9 : 3-(2-CYCLOPROPYLAMINO ETHYL) [3H] OXAZOLO[4,5-b] PYRIDIN-2-ONE (BROMHYDRATE)**

En procédant comme dans l'exemple 8, mais en remplaçant l'isopropylamine par la cyclopropylamine, on obtient le produit du titre.

**EXEMPLE 10 : 3-(2-DIETHYLAMINO ETHYL)[3H] OXAZOLO [4,5-b] PYRIDIN-2-ONE (OXALATE)**

En procédant comme dans l'exemple 1, mais en remplaçant la pipéridine par la diéthylamine, on obtient le produit du titre sous forme de base. Dissoudre dans l'éthanol et ajouter 0,01 mole d'acide oxalique. Essorer. Sécher. Recristalliser.

Point de fusion : 139°C

**EXEMPLE 11 : 3-[2-(N METHYL N CYCLOHEXYLAMINO)ETHYL][3H] OXAZOLO [4,5-b] PYRIDIN-2-ONE**

En procédant comme dans l'exemple 1, mais en remplaçant la pipéridine par la N méthyl N cyclohexylamine, on obtient le produit du titre.

**EXEMPLE 12 : 3-[2-(4-PHENYL PIPERIDINO)ETHYL]-[3H] OXAZOLO [4,5-b] PYRIDIN-2-ONE**

En procédant comme dans l'exemple 1, mais en remplaçant la pipéridine par la 4-phényl pipéridine, on obtient le produit du titre.

Point de fusion : 88°C

**EXEMPLE 13 : 3-[2-(1,2,3,4-TETRAHYDROQUINOL-1-YL) ETHYL][3H]OXAZOLO[4,5-b] PYRIDIN-2-ONE**

En procédant comme dans l'exemple 1, mais en remplaçant la pipéridine par la tétrahydro 1.2.3.4.quinoléine, on obtient le produit du titre.

**EXEMPLE 14 : 3-MORPHOLINOMETHYL[3H]OXAZOLO [4,5-b] PYRIDIN-2-ONE**

Dissoudre 4,1 g (0,03 mole) de [3H] oxazolo [4,5-b] pyridinone - 2 dans 100 ml d'alcool à 95 °C. Ajouter 2,88 g (0,33 mole) de morpholine puis 3 ml d'une solution aqueuse de formol à 30 %. Agiter au bain-marie à une température voisine de 50 °C pendant une heure trente en maintenant l'agitation. Laisser reposer une heure à température ambiante. Essorer les cristaux et recristalliser.

Rendement : 85 %

Caractéristiques spectrales :

Infrarouge : 3100-2700 $cm^{-1}$ ν (CH)

1760 $cm^{-1}$ ν (CO)
1590 $cm^{-1}$ ν (C=C)

**EXEMPLE 15 : 3-(PYRROLIDIN-1-YL METHYL) [3H]OXAZOLO [4,5-b] PYRIDIN-2-ONE**

En procédant comme dans l'exemple 14, mais en remplaçant la morpholine par la pyrrolidine, on obtient le produit du titre.

**EXEMPLE 16 : 3-PIPERIDINOMETHYL [3H] OXAZOLO [4,5-b]PYRIDIN-2-ONE**

En procédant comme dans l'exemple 14, mais en remplaçant la morpholine par la pipéridine, on obtient le produit du titre.

**EXEMPLE 17 : 3-(2-PIPERIDINO ETHYL)5-METHYL [3H] OXAZOLO [4,5-b] PYRIDIN-2-ONE**

En procédant comme dans l'exemple 1, mais en remplaçant la 3-(2-bromo éthyl)[3H] oxazolo [4,5-b] pyridin-2-one par la 3-(2-bromo éthyl)5-méthyl [3H] oxazolo [4,5-b] pyridin-2-one obtenue dans la préparation 6, on obtient le produit du titre.

**EXEMPLE 18 : 3-[2-(4-TRIFLUOROMETHYL BENZYLAMINO) ETHYL][3H] OXAZOLO [4,5-b] PYRIDIN-2-ONE**

En procédant comme dans l'exemple 1, mais en remplaçant la pipéridine par la 4-trifluorométhyl benzylamine, on obtient le produit du titre.

**EXEMPLE 19 : 3-[2-(4-METHYL PIPERIDINO) ETHYL][3H] OXAZOLO [4,5-b] PYRIDIN-2-ONE**

En procédant comme dans l'exemple 1, mais en remplaçant la pipéridine par la 4-méthyl pipéridine, on obtient le produit du titre.

**EXEMPLE 20 : 3-[2-(4-BENZYL PIPERIDINO) ETHYL][3H] OXAZOLO [4,5-b] PYRIDIN-2-ONE**

En procédant comme dans l'exemple 1, mais en remplaçant la pipéridine par la 4-benzyl pipéridine, on obtient le produit du titre.

**EXEMPLE 21 : 3-[2-(2-CHLOROETHYL AMINO) ETHYL][3H] OXAZOLO [4,5-b] PYRIDIN-2-ONE**

En procédant comme dans l'exemple 1, mais en remplaçant la pipéridine par la 2-chloro éthylamine, on obtient le produit du titre.

**EXEMPLE 22 : 3-[2-[4-(4.4'-DIFLUOROBENZHYDRYL)PIPERAZIN-1-YL] ETHYL] [3H] OXAZOLO [4,5-b] PYRIDIN-2-ONE**

En procédant comme dans l'exemple 1 mais en remplaçant la pipéridine par la 1-(4,4'-difluoro benzhydryl)pipérazine, on obtient le produit du titre.

**EXEMPLE 23 : 3-[2-(4-BENZHYDRYL PIPERAZIN-1-YL)ETHYL] [3H] OXAZOLO [4,5-b] PYRIDIN-2-ONE**

En procédant commes dans l'exemple 1 mais en remplaçant la pipéridine par la 1-benzhydryl pipérazine, on obtient le produit du titre.

**EXEMPLE 24 : 3-[2-[4-(4-CHLORO BENZHYDRYL)PIPERAZIN-1-YL) ETHYL] [3H] OXAZOLO [4,5-b] PYRIDIN-2-ONE**

En procédant comme dans l'exemple 1 mais en remplaçant la pipéridine par la 1-(4-chloro benzhydryl)pipérazine, on obtient le produit du titre.

### EXEMPLE 25 : 2-(2-PIPERIDINOETHYLAMINO)PYRIDIN-3-OL

Placer 0,01 mole de 3-(2-pipéridinoéthyl)[3H] oxazolo [4,5-b] pyridin-2-one obtenue dans l'exemple 1 dans 50 ml de solution d'hydroxyde de sodium à 10%. Chauffer à reflux quatre heures sous agitation magnétique. Après refroidissement, la solution est acidifiée par de l'acide chlorhydrique à 30%. Ajouter tout en refroidissant une solution aqueuse saturée de bicarbonate de sodium jusqu'à Ph=7. Le précipité est filtré et lavé trois fois à l'eau, séché sous vide dans un dessicateur puis relavé.
En procédant comme indiqué dans l'exemple 25, mais en utilisant comme matière première les composés obtenus dans les exemples 2 à 24 on obtient les 2-(amino(substitués) alkylamino) pyridin-3-ols éventuellement substitués.

## ETUDE PHARMACOLOGIQUE DES DERIVES DE L'INVENTION

### EXEMPLE 26 : ETUDE DE LA TOXICITE AIGUE

La toxicité a été appréciée après administration orale à des lots de cinq souris (20 ± 2 grammes) de doses croissantes (0,1-0,25-0,50-0,75-1 g (kg)). Les animaux ont été observés à intervalles réguliers au cours de la première journée et quotidiennement pendant les deux semaines suivant le traitement.
Il apparait que les composés de l'invention sont totalement atoxiques.

### EXEMPLE 27 : ETUDE DE L'ACTIVITE ANALGESIQUE

L'activité sur la douleur a été recherchée chez la souris (23-25 g) selon un protocole dérivé de la technique décrite par SIEGMUND (SIEGMUND E.A, R.A. CADMUS & GOLU, J. Pharm. Exp. Ther. 119, 184, 1957). Les souris réparties par randomisation en lots de 12 animaux, ont reçu le traitement par voie orale (excipient pour les témoins) 1 heure avant l'injection intra-péritonéale d'une solution hydroalcoolique de phényl-p-benzoquinone à 0,02 %. Les étirements sont dénombrés entre la 5ème et 10ème minute après l'injection.

Le pourcentage d'activité obtenu a été évalué pour chaque dose (% de diminution du nombre d'étirements chez les traités par rapport aux témoins). Une $ED_{50}$, dose entraînant une activité de 50 %, a été déterminée pour chaque produit testé.

### EXEMPLE 28 : ETUDE DE L'ACTIVITE ANTI-INFLAMMATOIRE

Le potentiel anti-inflammatoire des composés a été recherché sur un modèle d'inflammation aiguë provoquée par injection sous-cutanée d'une solution de carragénine au niveau de la patte postérieure de rat, selon une technique inspirée de la méthode de WINTER C.H, E.A. RISLEY, G.N. NUSS - Proc. Soc. Exp. Med. 111, 554, 1962). Les rats (100-120 g) randomisés en lot de 8, ont été traités (y compris les témoins qui reçoivent l'excipient) 1 heure avant l'injection locale d'une suspension à 0,5 % de carragénine (type IV, Sigma ; 0,1 ml par rat). L'oedème est déterminé 3 heures après l'injection, par mesure pléthysmométrique (pléthysmomètre à eau UGO BASILE) du volume de chacune des pattes postérieures (oedème = volume patte enflammée - volume patte non enflammée).

Le pourcentage d'activité correspond au pourcentage de diminution de l'oedème moyen du lot comparativement à la moyenne du lot témoin correspondant. Une $ED_{30}$ dose entraînant une activité de 30 % a été déterminée.

Cette $ED_{30}$ est égale à 50 $mg.kg^{-1}$ pour le composé de l'exemple 6 du brevet US 4 038 396. Elle est très nettement supérieure à cette valeur pour tous les composés de l'invention jusqu'à une dose de 150 $mg.kg^{-1}$, ils n'ont pas d'activité anti-inflammatoire.

### EXEMPLE 29 : TEST DE TOLERANCE GASTRIQUE

On soumet au jeûne pendant 24 heures un groupe de 5 rats. Les produits à tester sont administrés per os, en suspension dans du sirop de gomme à une dose de 150 $mg.kg^{-1}$. Après administration les animaux sont placés dans des conditions dites de semi contrainte réputées pour être ulcérigènes (ils sont enfermés dans des cages étroites). Au bout de 6 heures, ils sont sacrifiés et la paroi stomacale examinée. On définit la tolérance gastrique à partir de la cotation de SHAY et LAMBLING. La gravité des zones irritées et des points d'ulcération est notée de 0 à 3. On définit un indice d'ulcération U et un indice d'hyperémie ou irritation H.

$$U \text{ ou } H = \frac{\text{(Somme des cotations) x (nombre de points d'estomac testés)}}{\text{Nombre d'animaux étudiés}}$$

Indice global d'agressivité G = 3U + H.
Cet indice G vaut 1 pour les produits de l'invention, 12 pour le témoin et 74 pour l'aspirine.

**EXEMPLE 30 : TEST DE FIXATION AUX RECEPTEURS**

L'étude de la fixation des composés de l'invention à différentes catégories de récepteurs a été réalisée selon des techniques conventionnelles. Il apparaît que certains composés de l'invention se lient avec une bonne affinité ($10^{-7}$M) aux récepteurs mécaniques $M_1$ ce qui ne semble pas être rapporté à ce jour pour des structures comparables.

**EXEMPLE 31 : COMPOSITON PHARMACEUTIQUE : COMPRIME**

Comprimés dosés à 25 mg de 3-(2-pipéridino éthyl) [3H] oxazolo [4,5-b] pyridinone - 2
Formule de préparation pour 1000 comprimés.

| | |
|---|---|
| 3-[2-(1 pyrrolidinyl éthyl)][3H] oxazolo [4,5-b] pyridin-2-one . . | 25 g |
| Amidon de blé . . . . . . . . . . . . . . . . . . . . . . . . | 15 g |
| Amidon de maïs . . . . . . . . . . . . . . . . . . . . . . . . | 15 g |
| Lactose . . . . . . . . . . . . . . . . . . . . . . . . . . . | 65 g |
| Stéarate de magnésium . . . . . . . . . . . . . . . . . . . . | 1 g |
| Silice . . . . . . . . . . . . . . . . . . . . . . . . . . . . | 1 g |
| Hydroxypropylcellulose . . . . . . . . . . . . . . . . . . . . | 2 g |

**ANNEXE**

Nomenclature

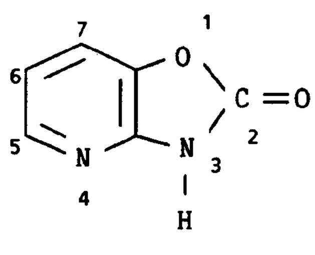

**[3H] OXAZOLO [4,5b] PYRIDIN-2-ONE**

**Revendications**

**1/** Composés de formule générale (I) :

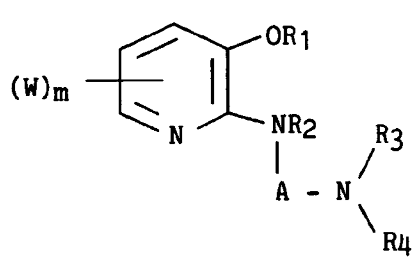

(I)

dans laquelle :

$R_1$ et $R_2$ représentent chacun un atome d'hydrogène ou forment avec l'azote et l'oxygène qui les portent, un chaînon - O - CO - N,

W représente un atome d'halogène ou un groupement alkyle ou alkoxy inférieur éventuellement substitué par un ou plusieurs atomes d'halogène tel que trifluorométhyle, et m étant compris entre 0 et 3,

A est un radical alkyle linéaire ou ramifié comprenant de 1 à 6 atomes de carbone,

$R_3$ et $R_4$ identiques ou différents représentent :

– un atome d'hydrogène,
– un groupement alkyle inférieur linéaire ou ramifié,
– un groupement alkényle inférieur linéaire ou ramifié,
– un groupement cycloalkyle de 6 à 10 atomes de carbone,
– un groupement aryle ou alkyle inférieur aryle,

chacun de ces groupements étant éventuellement substitué par un ou plusieurs atomes d'halogène ou groupements trifluorométhyle, hydroxy ou alkoxy inférieurs,

ou bien :

$R_3$ et $R_4$ constituent avec l'atome d'azote auquel ils sont liés un système hétérocyclique azoté mono ou bicyclique, saturé ou non comprenant un maximum de 12 atomes - non compris les atomes d'hydrogène - parmi lesquels on peut compter un à trois hétéroatomes choisis parmi azote, oxygène ou soufre substitué ou non par un groupement alkyle inférieur ou phényle ou phénylalkyle inférieur ou diphénylalkyle inférieurs, les groupements phényle, phénylalkyle inférieur ou diphénylalkyl inférieur pouvant être substitués par un ou plusieurs atomes d'halogène ou groupements hydroxy, alkyle inférieurs, alkoxy inférieurs ou trifluorométhyle, à la condition que $R_3$ et $R_4$ ne constituent pas avec l'atome d'azote auquel ils sont liés un système aryl-1 pipérazinique ou hétéroaryl-1 pipérazinique,

étant entendu que par radical alkyle inférieur, alkényle inférieur, ou alkyloxy inférieur, on entend un groupement linéaire ou ramifié comprenant de 1 à 6 atomes de carbone et que par groupements aryle ou hétéroaryle, on entend des groupements mono ou bicycliques insaturés comprenant de 5 à 12 atomes - non compris les atomes d'hydrogène - intégrant ou non dans leur squelette carboné un, deux ou trois hétéroatomes choisis parmi azote, oxygène ou soufre,

leurs isomères, épimères, diastéréoisomères,

ainsi que leurs sels d'addition à un acide pharmaceutiquement acceptable et lorsque $R_1$ et $R_2$ représentent chacun un atome d'hydrogène, leurs sels d'addition à une base pharmaceutiquement acceptable.

**2/** Composés selon la revendication 1 pour lesquels $R_1$ et $R_2$ forment ensemble un groupement CO, de formule (I/A) :

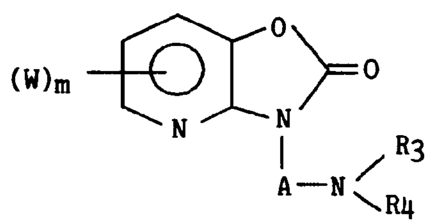

**(I/A)**

leurs isomères ainsi que leurs sels d'addition à un acide pharmaceutiquement acceptable.

**3/** Composés selon la revendication 1 pour lesquels $R_1$ et $R_2$ représentent chacun un atome d'hydrogène de formule (I/B) :

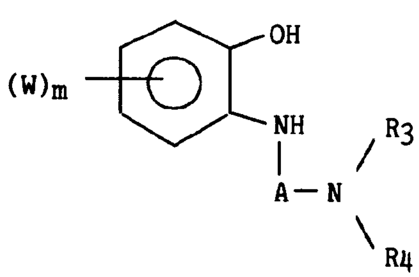

**(I/B)**

leurs isomères ainsi que leurs sels d'addition à un acide pharmaceutiquement acceptable ou à une base pharmaceutiquement acceptable.

**4/** Composés selon la revendication 1 pour lesquels $R_3$ et $R_4$ représentent avec l'atome d'azote qui les porte un système hétérocyclique azoté mono ou bicyclique saturé comprenant de 5 à 12 atomes - non compris les atomes d'hydrogène - intégrant ou non dans leur squelette carboné un, deux ou trois hétéroatomes choisis parmi azote, oxygène ou soufre,
leurs isomères, épimères, diastéréoisomères,
ainsi que leurs sels d'addition à un acide pharmaceutiquement acceptable et lorsque $R_1$ et $R_2$ représentent chacun un atome d'hydrogène, leurs sels d'addition à une base pharmaceutiquement acceptable.

**5/** Composé selon l'une des revendication 1 et 2 qui est la 3-(2-pipéridinoéthyl) [3H] oxazolo [4,5-b] pyridine-2-one ainsi que ses sels d'addition à un acide pharmaceutiquement acceptable.

**6/** Composé selon l'une des revendications 1 et 2 qui est la 3-[2-(4-méthyl pipérazin-1-yl)éthyl] [3H] oxazolo [4,5-b] pyridine-2-one ainsi que ses sels d'addition à un acide pharmaceutiquement acceptable.

**7/** Composé selon l'une des revendications 1 et 2 qui est la 3-[2-(1-pyrrolidinyl)éthyl] [3H] oxazolo [4,5-b] pyridine-2-one ainsi que ses sels d'addition à un acide pharmaceutiquement acceptable.

**8/** Composé selon l'une des revendications 1 et 2 qui est la 3-(2-morpholinoéthyl) [3H] oxazolo [4,5-b] pyridine-2-one ainsi que ses sels d'addition à un acide pharmaceutiquement acceptable.

**9/** Composé selon l'une des revendications 1 et 3 qui est le 2-(2-pipéridinoéthylamino) pyridin-3-ol ainsi que ses sels d'addition à un acide ou une base pharmaceutiquement acceptable.

**10/** Procédé d'obtention de composés de formule (I) selon la revendication 1, caractérisé en ce que l'on fait réagir un dérivé de formule (II) :

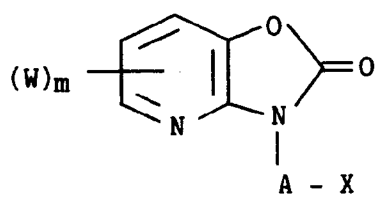

**(II)**

dans lequel W, m et A ont la même signification que dans la formule (I) et X représente un atome d'halogène, préférentiellement sous atmosphère inerte avec un dérivé de formule (III), préférentiellement en excès :

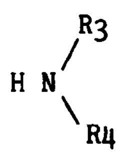

**(III)**

dans laquelle $R_3$ et $R_4$ ont la même signification que dans la formule (I),
en milieu organique en présence d'un agent basique et à température comprise entre la température ambiante et la température du reflux du solvant choisi pour conduire après refroidissement, extraction et éventuelle purification à un dérivé de formule (I/A) :

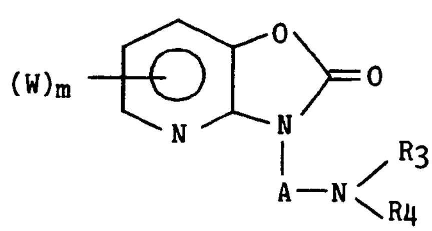

**(I/A)**

cas particulier des dérivés de formule (I) pour lesquels $R_1$ forme avec $R_2$ un groupement C = O,
que l'on peut, si on le désire, séparer le cas échéant, en ses isomères puis salifier par un acide pharmaceutiquement acceptable,
dérivé de formule (I/A) que l'on peut traiter, si on le désire, par un agent alcalin en solution aqueuse, à une température comprise entre la température ambiante et la température d'ébullition du milieu réactionnel pour conduire, après éventuelle acidification et/ou neutralisation du milieu réactionnel à un dérivé de formule (I/B) :

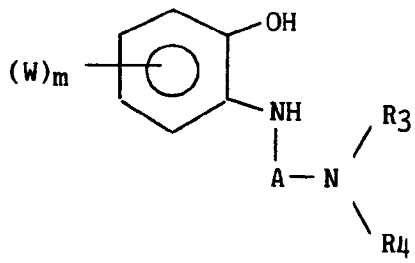

(I/B)

cas particulier des dérivés de formule (I) pour lesquels $R_1$ et $R_2$ représentent chacun un atome d'hydrogène et W, m, A, $R_3$ et $R_4$ ont la même signification que précédemment, que l'on purifie, si nécessaire par une technique choisie parmi cristallisation ou chromatographie et que l'on salifie, si on le désire par un acide ou une base pharmaceutiquement acceptables.

**11/** Procédé de préparation de composés de formule (I/A) selon la revendication 2 caractérisé en ce que l'on fait réagir un dérivé de formule (V) :

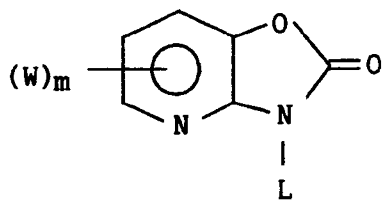

(V)

dans laquelle W et m ont la même signification que dans la formule (I) et L représente un métal alcalin, avec un dérivé de formule (VII) :

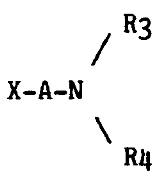

(VII)

dans laquelle A, $R_3$ et $R_4$ ont la même définition que dans la formule (I) et X représente un atome d'halogène en milieu organique à température comprise entre la température ambiante et la température de reflux du solvant choisi pour conduire, après éventuels refroidissement, extraction et purification à un dérivé de formule (I/A) dont on sépare le cas échéant les isomères et que l'on salifie si on le désire, par un acide pharmaceutiquement acceptable.

**12/** Procédé de préparation de composés de formule (I/A) selon la revendication 2 pour lesquels A représente un groupement $CH_2$, caractérisé en ce que l'on dissout en milieu d'alcool aliphatique inférieur, d'une part un dérivé de formule (IV) :

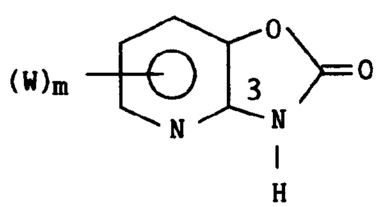

(IV)

dans laquelle W et m ont la même signification que dans la formule (I), d'autre part un léger excès d'une amine de formule (III) :

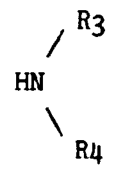

(III)

dans laquelle $R_3$ et $R_4$ ont la même signification que dans la formule (I),
et d'autre part un excès de formol,
pour conduire, après chauffage de la solution obtenue à une température comprise entre la température ambiante et la température d'ébullition, éventuels refroidissement, repos d'une à deux heures, filtration et chromatographie sur colonne de silice, à un dérivé de formule (I/A1) :

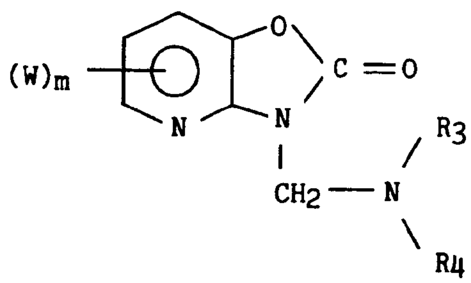

(I/A1)

cas particulier des dérivés de formule (I/A) dans laquelle W, m, $R_3$ et $R_4$ ont la même signification que dans la formule (I), et A représente un groupement $CH_2$,
que l'on peut salifier si on le désire par un acide pharmaceutiquement acceptable.

**13/** Procédé de préparation des composés de formule (I/A) selon la revendication 2 caractérisé en ce que l'on soumet à réaction un dérivé de formule (II) :

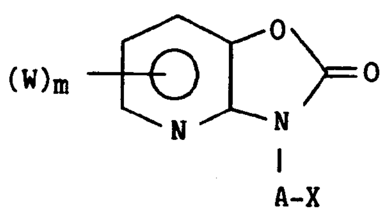

(II)

dans laquelle W, m et A ont la même signification que dans la formule (I) et X représente un atome d'halogène, que l'on condense, préférentiellement sous atmosphère inerte avec un dérivé de formule (III), préférentiellement en excès :

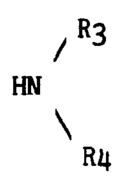

(III)

dans laquelle $R_3$ et $R_4$ ont la même définition que dans la formule (I),
en milieu organique en la présence éventuelle d'un excès d'un agent basique et à température comprise entre l'ambiante et la température du reflux du solvant choisi pour conduire après éventuels refroidissement, extraction et purification à un dérivé de formule (I/A) :

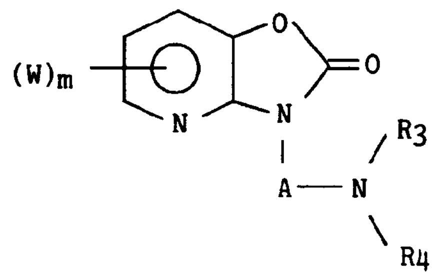

(I/A)

dans laquelle W, m, A, $R_3$ et $R_4$ ont la même définition que dans la formule (I), que l'on peut, si nécessaire, séparer en ses isomères et si on le désire, salifier par un acide pharmaceutiquement acceptable.

**14/** Procédé de préparation de composé de formule (I/B) selon la revendication 3 caractérisé en ce que l'on soumet un composé de formule (I/A) :

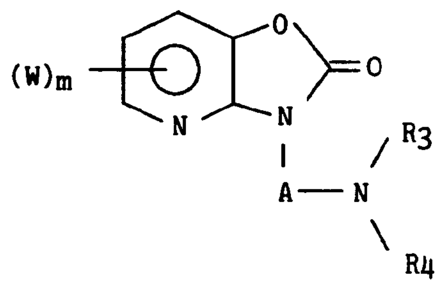

(I/A)

dans laquelle W, m, A, $R_3$ et $R_4$ ont la même signification que dans la formule (I), ou l'un de ses sels d'addition à un acide pharmaceutiquement acceptables à un agent alcalin en solution aqueuse, à température comprise entre la température ambiante et la température d'ébullition du milieu réactionnel pour conduire, après neutralisation ou acidification du milieu réactionnel à un composé de formule (I/B) :

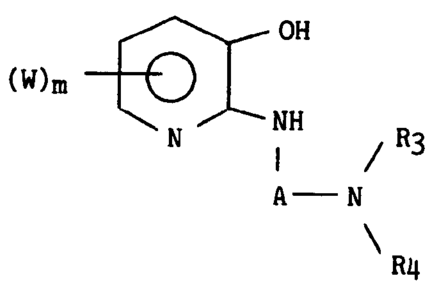

(I/B)

dans laquelle W, m, A, $R_3$ et $R_4$ ont la même définition que dans la formule (I), que l'on salifie, si on le souhaite, par un acide ou une base pharmaceutiquement acceptable après éventuelle purification.

**15/** Composition pharmaceutique contenant comme principe actif au moins un composé selon l'une des revendications 1 à 9 en combinaison avec un ou plusieurs excipients ou véhicules inertes, non-toxiques, pharmaceutiquement acceptables.

**16/** Composition pharmaceutique selon la revendication 15 contenant au moins un principe actif selon l'une des revendications 1 à 9 utilisables dans le traitement d'algies et des troubles de l'insuffisance circulatoire cérébrale.

**Revendications pour l'Etat contractant suivant: GR**

**1/** Procédé de préparation de composés de formule générale (I) :

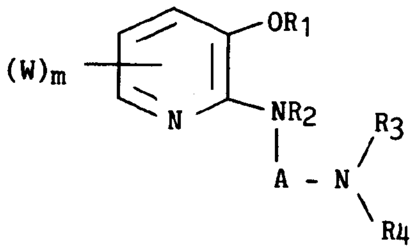

(I)

dans laquelle :

$R_1$ et $R_2$ représentent chacun un atome d'hydrogène ou forment avec l'azote et l'oxygène qui les portent, un chaînon - O - CO - N,

W représente un atome d'halogène ou un groupement alkyle ou alkoxy inférieur éventuellement substitué par un ou plusieurs atomes d'halogène tel que trifluorométhyle, et m étant compris entre 0 et 3,

A est un radical alkyle linéaire ou ramifié comprenant de 1 à 6 atomes de carbone,

$R_3$ et $R_4$ identiques ou différents représentent :

– un atome d'hydrogène,
– un groupement alkyle inférieur linéaire ou ramifié,
– un groupement alkényle inférieur linéaire ou ramifié,
– un groupement cycloalkyle de 6 à 10 atomes de carbone,
– un groupement aryle ou alkyle inférieur aryle,

chacun de ces groupements étant éventuellement substitué par un ou plusieurs atomes d'halogène ou groupements trifluorométhyle, hydroxy ou alkoxy inférieurs,

ou bien :

$R_3$ et $R_4$ constituent avec l'atome d'azote auquel ils sont liés un système hétérocyclique azoté mono ou bicyclique, saturé ou non comprenant un maximum de 12 atomes - non compris les atomes d'hydrogène - parmi lesquels on peut compter un à trois hétéroatomes choisis parmi azote, oxygène ou soufre substitué ou non par un groupement alkyle inférieur ou phényle ou phénylalkyle inférieur ou diphénylalkyle inférieurs, les groupements phényle, phénylalkyle inférieur ou diphénylalkyl inférieur pouvant être substitués par un ou plusieurs atomes d'halogène ou groupements hydroxy, alkyle inférieurs, alkoxy inférieurs ou trifluorométhyle, à la condition que $R_3$ et $R_4$ ne constituent pas avec l'atome d'azote auquel ils sont liés un système aryl-1 pipérazinique ou hétéroaryl-1 pipérazinique,

étant entendu que par radical alkyle inférieur, alkényle inférieur, ou alkyloxy inférieur, on entend un groupement linéaire ou ramifié comprenant de 1 à 6 atomes de carbone et que par groupements aryle ou hétéroaryle, on entend des groupements mono ou bicycliques insaturés comprenant de 5 à 12 atomes - non compris les atomes d'hydrogène - intégrant ou non dans leur squelette carboné un, deux ou trois hétéroatomes choisis parmi azote, oxygène ou soufre,

leurs isomères, épimères, diastéréoisomères,

ainsi que leurs sels d'addition à un acide pharmaceutiquement acceptable et lorsque $R_1$ et $R_2$ représentent chacun un atome d'hydrogène, leurs sels d'addition à une base pharmaceutiquement acceptable,

caractérisé en ce que l'on fait réagir un dérivé de formule (II) :

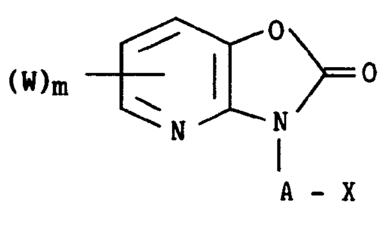

(II)

dans lequel W, m et A ont la même signification que dans la formule (I) et X représente un atome d'halogène, préférentiellement sous atmosphère inerte avec un dérivé de formule (III), préférentiellement en excès :

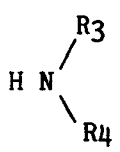

(III)

dans laquelle $R_3$ et $R_4$ ont la même signification que dans la formule (I), en milieu organique en présence d'un agent basique et à température comprise entre la température ambiante et la température du reflux du solvant choisi pour conduire après refroidissement, extraction et éventuelle purification à un dérivé de formule (I/A) :

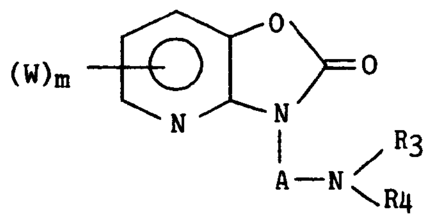

(I/A)

cas particulier des dérivés de formule (I) pour lesquels $R_1$ forme avec $R_2$ un groupement C = O, que l'on peut, si on le désire, séparer le cas échéant, en ses isomères puis salifier par un acide pharmaceutiquement acceptable,
dérivé de formule (I/A) que l'on peut traiter, si on le désire, par un agent alcalin en solution aqueuse, à une température comprise entre la température ambiante et la température d'ébullition du milieu réactionnel pour conduire, après éventuelle acidification et/ou neutralisation du milieu réactionnel à un dérivé de formule (I/B) :

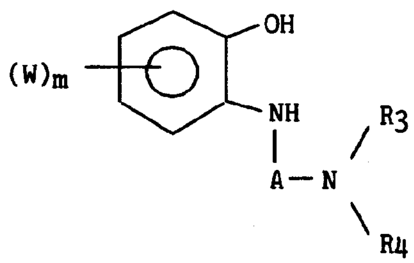

(I/B)

cas particulier des dérivés de formule (I) pour lesquels $R_1$ et $R_2$ représentent chacun un atome d'hydrogène et W, m, A, $R_3$ et $R_4$ ont la même signification que précédemment, que l'on purifie, si nécessaire par une technique choisie parmi cristallisation ou chromatographie et que l'on salifie, si on le désire par un acide ou une base pharmaceutiquement acceptables.

**2/** Procédé de préparation selon la revendication 1 de composés de formule (I/A) caractérisé en ce que l'on fait réagir un dérivé de formule (V) :

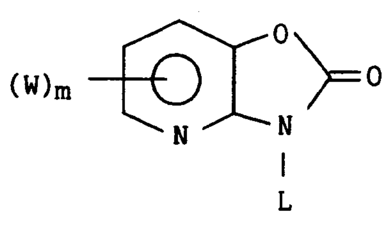

(V)

dans laquelle W et m ont la même signification que dans la formule (I) et L représente un métal alcalin, avec un dérivé de formule (VII) :

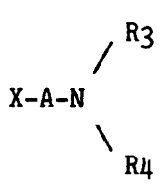

(VII)

dans laquelle A, $R_3$ et $R_4$ ont la même définition que dans la formule (I) et X représente un atome d'halogène en milieu organique à température comprise entre la température ambiante et la température de reflux du solvant choisi pour conduire, après éventuels refroidissement, extraction et purification à un dérivé de formule (I/A) dont on sépare le cas échéant les isomères et que l'on salifie si on le désire, par un acide pharmaceutiquement acceptable.

**3/** Procédé de préparation selon la revendication 1 des composés de formule (I/A) pour lesquels A représente un groupement $CH_2$, caractérisé en ce que l'on dissout en milieu d'alcool aliphatique inférieur, d'une part un dérivé de formule (IV) :

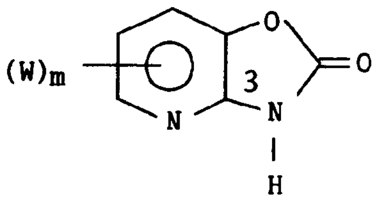

(IV)

dans laquelle W et m ont la même signification que dans la formule (I), d'autre part un léger excès d'une amine de formule (III) :

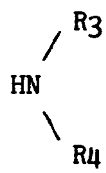

(III)

dans laquelle $R_3$ et $R_4$ ont la même signification que dans la formule (I), et d'autre part un excès de formol, pour conduire, après chauffage de la solution obtenue à une température comprise entre la température ambiante et la température d'ébullition, éventuels refroidissement, repos d'une à deux heures, filtration et chromatographie sur colonne de silice, à un dérivé de formule (I/A1) :

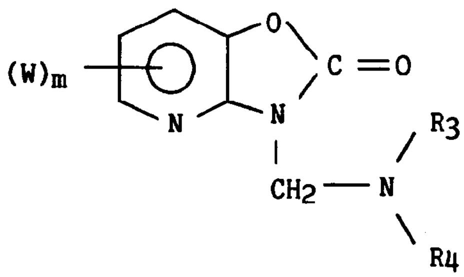

(I/A1)

cas particulier des dérivés de formule (I/A) dans laquelle W, m, $R_3$ et $R_4$ ont la même signification que dans la formule (I), et A représente un groupement $CH_2$, que l'on peut salifier si on le désire par un acide pharmaceutiquement acceptable.

**4/** Procédé de préparation selon la revendication 1 des composés de formule (I/A) caractérisé en ce que l'on soumet à réaction un dérivé de formule (II) :

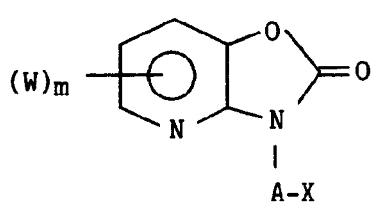

(II)

dans laquelle W, m et A ont la même signification que dans la formule (I) et X représente un atome d'halogène, que l'on condense, préférentiellement sous atmosphère inerte avec un dérivé de formule (III), préférentiellement en excès :

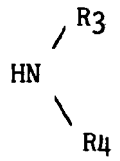

(III)

dans laquelle $R_3$ et $R_4$ ont la même définition que dans la formule (I), en milieu organique en la présence éventuelle d'un excès d'un agent basique et à température comprise entre l'ambiante et la température du reflux du solvant choisi pour conduire après éventuels refroidissement, extraction et purification à un dérivé de formule (I/A) :

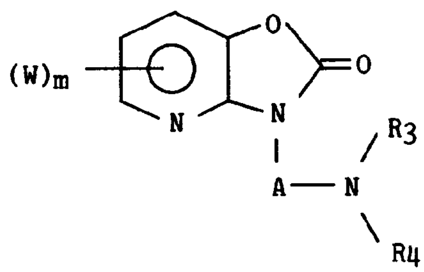

(I/A)

dans laquelle W, m, A, $R_3$ et $R_4$ ont la même définition que dans la formule (I), que l'on peut, si nécessaire, séparer en ses isomères et si on le désire, salifier par un acide pharmaceutiquement acceptable.

**5/** Procédé de préparation selon la revendication 1 de composé de formule (I/B) caractérisé en ce que l'on soumet un composé de formule (I/A) :

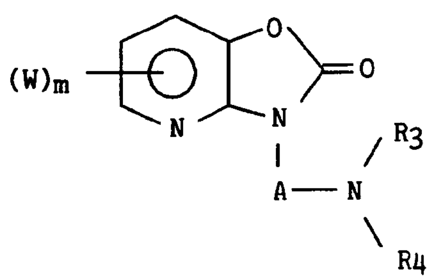

(I/A)

dans laquelle W, m, A, $R_3$ et $R_4$ ont la même signification que dans la formule (I), ou l'un de ses sels d'addition à un acide pharmaceutiquement acceptables à un agent alcalin en solution aqueuse, à température comprise entre la température ambiante et la température d'ébullition du milieu réactionnel pour conduire, après neutralisation ou acidification du milieu réactionnel à un composé de formule (I/B) :

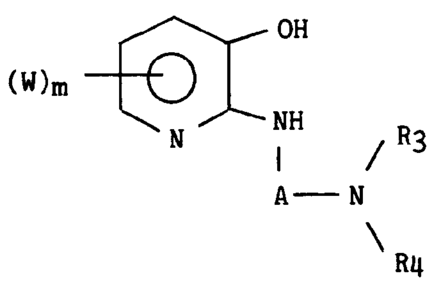

(I/B)

dans laquelle W, m, A, $R_3$ et $R_4$ ont la même définition que dans la formule (I), que l'on salifie, si on le souhaite, par un acide ou une base pharmaceutiquement acceptable après éventuelle purification.

**6/** Procédé de préparation selon la revendication 1 des composés pour lesquels $R_1$ et $R_2$ forment ensemble un groupement CO, de formule (I/A) :

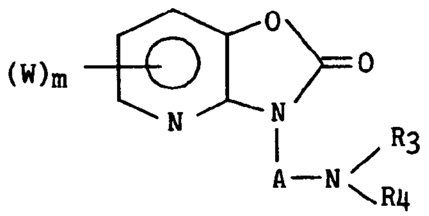

(I/A)

leurs isomères ainsi que leurs sels d'addition à un acide pharmaceutiquement acceptable.

**7/** Procédé de préparation selon la revendication 1 des composés pour lesquels $R_1$ et $R_2$ représentent chacun un atome d'hydrogène de formule (I/B) :

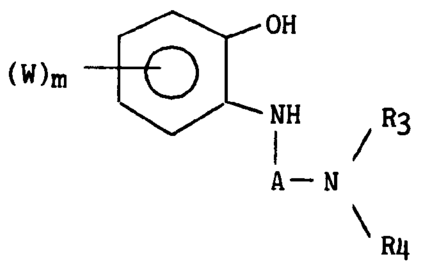

(I/B)

leurs isomères ainsi que leurs sels d'addition à un acide pharmaceutiquement acceptable ou à une base pharmaceutiquement acceptable.

**8/** Procédé de préparation selon la revendication 1 des composés pour lesquels $R_3$ et $R_4$ représentent avec l'atome d'azote qui les porte un système hétérocyclique azoté mono ou bicyclique saturé comprenant de 5 à 12 atomes - non compris les atomes d'hydrogène - intégrant ou non dans leur squelette carboné un, deux ou trois hétéroatomes choisis parmi azote, oxygène ou soufre,
leurs isomères, épimères, diastéréoisomères,
ainsi que leurs sels d'addition à un acide pharmaceutiquement acceptable et lorsque $R_1$ et $R_2$ représentent chacun un atome d'hydrogène, leurs sels d'addition à une base pharmaceutiquement acceptable.

**9/** Procédé de préparation selon la revendication 1 du composé qui est la 3-(2-pipéridinoéthyl) [3H] oxazolo [4,5-b] pyridine-2-one ainsi que ses sels d'addition à un acide pharmaceutiquement acceptable.

**10/** Procédé de préparation selon la revendication 1 du composé qui est la 3-[2-(4-méthyl pipérazin-1-yl)éthyl] [3H] oxazolo [4,5-b] pyridine-2-one ainsi que ses sels d'addition à un acide pharmaceutiquement acceptable.

**11/** Procédé de préparation selon la revendication 1 du composé qui est la 3-[2-(1-pyrrolidinyl)éthyl] [3H] oxazolo [4,5-b] pyridine-2-one ainsi que ses sels d'addition à un acide pharmaceutiquement acceptable.

**12/** Procédé de préparation selon la revendication 1 du composé qui est la 3-(2-morpholinoéthyl) [3H] oxazolo [4,5-b] pyridine-2-one ainsi que ses sels d'addition à un acide pharmaceutiquement acceptable.

**13/** Procédé de préparation selon la des revendication 1 du composé qui est le 2-(2- pipéridinoéthylamino) pyridin-3-ol ainsi que ses sels d'addition à un acide ou une base pharmaceutiquement acceptable.

**14/** Procédé de préparation de composition pharmaceutique contenant comme principe actif au moins un composé préparé selon l'une des revendications 1 à 13 en combinaison avec un ou plusieurs excipients ou véhicules inertes, non-toxiques, pharmaceutiquement acceptables.

**15/** Procédé de préparation de composition pharmaceutique selon la revendication 14 utilisables dans le traitement d'algies et des troubles de l'insuffisance circulatoire cérébrale.

**Revendications pour l'Etat contractant suivant: ES**

**1/** Procédé de préparation de composés de formule générale (I) :

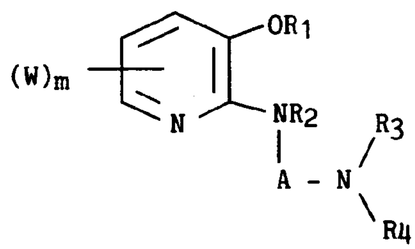

(I)

dans laquelle :

$R_1$ et $R_2$ représentent chacun un atome d'hydrogène ou forment avec l'azote et l'oxygène qui les portent, un chaînon - O - CO - N,

W représente un atome d'halogène ou un groupement alkyle ou alkoxy inférieur éventuellement substitué par un ou plusieurs atomes d'halogène tel que trifluorométhyle, et m étant compris entre 0 et 3,

A est un radical alkyle linéaire ou ramifié comprenant de 1 à 6 atomes de carbone,

$R_3$ et $R_4$ identiques ou différents représentent :

- un atome d'hydrogène,
- un groupement alkyle inférieur linéaire ou ramifié,
- un groupement alkényle inférieur linéaire ou ramifié,
- un groupement cycloalkyle de 6 à 10 atomes de carbone,
- un groupement aryle ou alkyle inférieur aryle,

chacun de ces groupements étant éventuellement substitué par un ou plusieurs atomes d'halogène ou groupements trifluorométhyle, hydroxy ou alkoxy inférieurs,

ou bien :

$R_3$ et $R_4$ constituent avec l'atome d'azote auquel ils sont liés un système hétérocyclique azoté mono ou bicyclique, saturé ou non comprenant un maximum de 12 atomes - non compris les atomes d'hydrogène - parmi lesquels on peut compter un à trois hétéroatomes choisis parmi azote, oxygène ou soufre substitué ou non par un groupement alkyle inférieur ou phényle ou phénylalkyle inférieur ou diphénylalkyle inférieurs, les groupements phényle, phénylalkyle inférieur ou diphénylalkyl inférieur pouvant être substitués par un ou plusieurs atomes d'halogène ou groupements hydroxy, alkyle inférieurs, alkoxy inférieurs ou trifluorométhyle, à la condition que $R_3$ et $R_4$ ne constituent pas avec l'atome d'azote auquel ils sont liés un système aryl-1 pipérazinique ou hétéroaryl-1 pipérazinique,

étant entendu que par radical alkyle inférieur, alkényle inférieur, ou alkyloxy inférieur, on entend un groupement linéaire ou ramifié comprenant de 1 à 6 atomes de carbone et que par groupements aryle ou hétéroaryle, on entend des groupements mono ou bicycliques insaturés comprenant de 5 à 12 atomes - non compris les atomes d'hydrogène - intégrant ou non dans leur squelette carboné un, deux ou trois hétéroatomes choisis parmi azote, oxygène ou soufre,

leurs isomères, épimères, diastéréoisomères,

ainsi que leurs sels d'addition à un acide pharmaceutiquement acceptable et lorsque $R_1$ et $R_2$ représentent chacun un atome d'hydrogène, leurs sels d'addition à une base pharmaceutiquement acceptable,

caractérisé en ce que l'on fait réagir un dérivé de formule (II) :

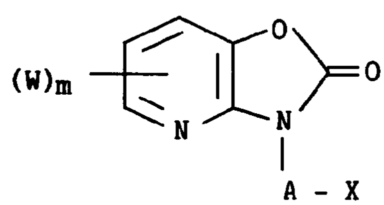

(II)

dans lequel W, m et A ont la même signification que dans la formule (I) et X représente un atome d'halogène, préférentiellement sous atmosphère inerte avec un dérivé de formule (III), préférentiellement en excès :

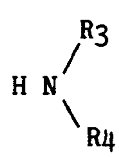

(III)

dans laquelle $R_3$ et $R_4$ ont la même signification que dans la formule (I), en milieu organique en présence d'un agent basique et à température comprise entre la température ambiante et la température du reflux du solvant choisi pour conduire après refroidissement, extraction et éventuelle purification à un dérivé de formule (I/A) :

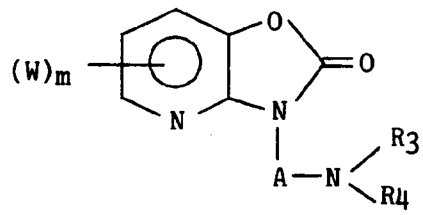

(I/A)

cas particulier des dérivés de formule (I) pour lesquels $R_1$ forme avec $R_2$ un groupement C = O, que l'on peut, si on le désire, séparer le cas échéant, en ses isomères puis salifier par un acide pharmaceutiquement acceptable,
dérivé de formule (I/A) que l'on peut traiter, si on le désire, par un agent alcalin en solution aqueuse, à une température comprise entre la température ambiante et la température d'ébullition du milieu réactionnel pour conduire, après éventuelle acidification et/ou neutralisation du milieu réactionnel à un dérivé de formule (I/B) :

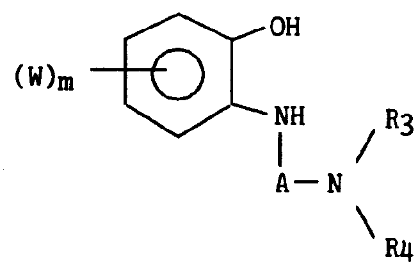

(I/B)

cas particulier des dérivés de formule (I) pour lesquels $R_1$ et $R_2$ représentent chacun un atome d'hydrogène et W, m, A, $R_3$ et $R_4$ ont la même signification que précédemment, que l'on purifie, si nécessaire par une technique choisie parmi cristallisation ou chromatographie et que l'on salifie, si on le désire par un acide ou une base pharmaceutiquement acceptables.

**2/** Procédé de préparation selon la revendication 1 de composés de formule (I/A) caractérisé en ce que l'on fait réagir un dérivé de formule (V) :

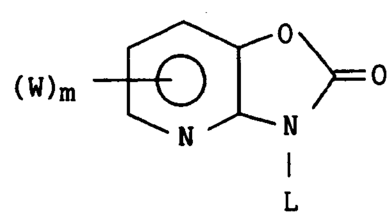

(V)

dans laquelle W et m ont la même signification que dans la formule (I) et L représente un métal alcalin, avec un dérivé de formule (VII) :

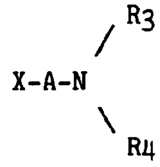

(VII)

dans laquelle A, $R_3$ et $R_4$ ont la même définition que dans la formule (I) et X représente un atome d'halogène en milieu organique à température comprise entre la température ambiante et la température de reflux du solvant choisi pour conduire, après éventuels refroidissement, extraction et purification à un dérivé de formule

(I/A) dont on sépare le cas échéant les isomères et que l'on salifie si on le désire, par un acide pharmaceutiquement acceptable.

**3/** Procédé de préparation selon la revendication 1 des composés de formule (I/A) pour lesquels A représente un groupement $CH_2$, caractérisé en ce que l'on dissout en milieu d'alcool aliphatique inférieur, d'une part un dérivé de formule (IV) :

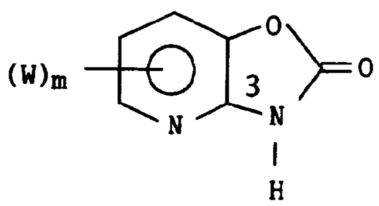

**(IV)**

dans laquelle W et m ont la même signification que dans la formule (I),
d'autre part un léger excès d'une amine de formule (III) :

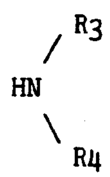

**(III)**

dans laquelle $R_3$ et $R_4$ ont la même signification que dans la formule (I),
et d'autre part un excès de formol,
pour conduire, après chauffage de la solution obtenue à une température comprise entre la température ambiante et la température d'ébullition, éventuels refroidissement, repos d'une à deux heures, filtration et chromatographie sur colonne de silice, à un dérivé de formule (I/A1) :

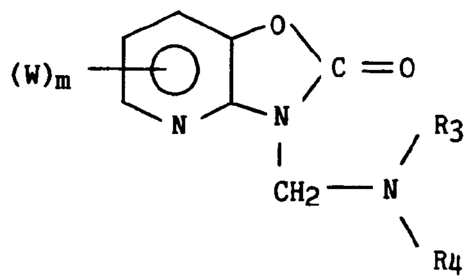

**(I/A1)**

cas particulier des dérivés de formule (I/A) dans laquelle W, m, $R_3$ et $R_4$ ont la même signification que dans la formule (I), et A représente un groupement $CH_2$,
que l'on peut salifier si on le désire par un acide pharmaceutiquement acceptable.

**4/** Procédé de préparation selon la revendication 1 des composés de formule (I/A) caractérisé en ce que l'on soumet à réaction un dérivé de formule (II) :

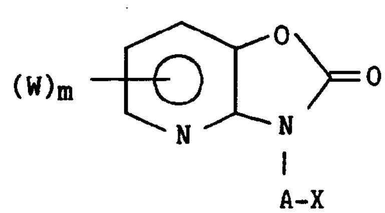

**(II)**

dans laquelle W, m et A ont la même signification que dans la formule (I) et X représente un atome d'halogène, que l'on condense, préférentiellement sous atmosphère inerte avec un dérivé de formule (III), préférentiellement en excès :

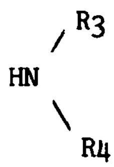

(III)

dans laquelle $R_3$ et $R_4$ ont la même définition que dans la formule (I), en milieu organique en la présence éventuelle d'un excès d'un agent basique et à température comprise entre l'ambiante et la température du reflux du solvant choisi pour conduire après éventuels refroidissement, extraction et purification à un dérivé de formule (I/A) :

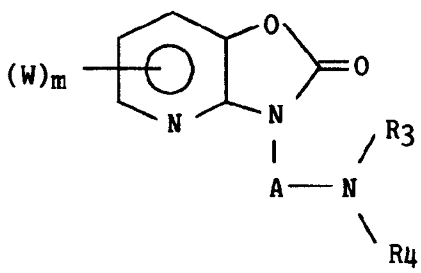

(I/A)

dans laquelle W, m, A, $R_3$ et $R_4$ ont la même définition que dans la formule (I), que l'on peut, si nécessaire, séparer en ses isomères et si on le désire, salifier par un acide pharmaceutiquement acceptable.

**5/** Procédé de préparation selon la revendication 1 de composé de formule (I/B) caractérisé en ce que l'on soumet un composé de formule (I/A) :

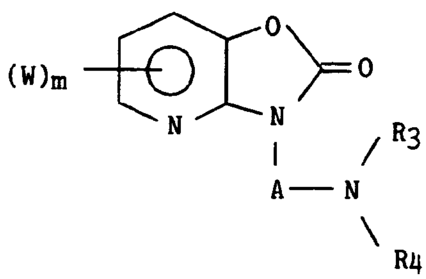

(I/A)

dans laquelle W, m, A, $R_3$ et $R_4$ ont la même signification que dans la formule (I), ou l'un de ses sels d'addition à un acide pharmaceutiquement acceptables à un agent alcalin en solution aqueuse, à température comprise entre la température ambiante et la température d'ébullition du milieu réactionnel pour conduire, après neutralisation ou acidification du milieu réactionnel à un composé de formule (I/B) :

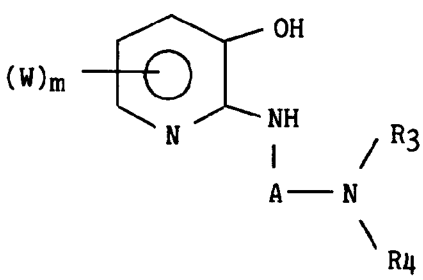

(I/B)

dans laquelle W, m, A, $R_3$ et $R_4$ ont la même définition que dans la formule (I), que l'on salifie, si on le souhaite, par un acide ou une base pharmaceutiquement acceptable après éventuelle purification.

**6/** Procédé de préparation selon la revendication 1 des composés pour lesquels $R_1$ et $R_2$ forment ensemble un groupement CO, de formule (I/A) :

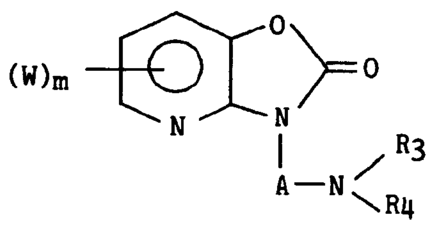

(I/A)

leurs isomères ainsi que leurs sels d'addition à un acide pharmaceutiquement acceptable.

**7/** Procédé de préparation selon la revendication 1 des composés pour lesquels $R_1$ et $R_2$ représentent chacun un atome d'hydrogène de formule (I/B) :

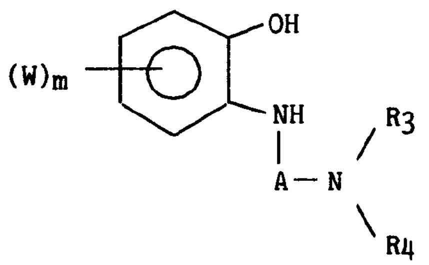

(I/B)

leurs isomères ainsi que leurs sels d'addition à un acide pharmaceutiquement acceptable ou à une base pharmaceutiquement acceptable.

**8/** Procédé de préparation selon la revendication 1 des composés pour lesquels $R_3$ et $R_4$ représentent avec l'atome d'azote qui les porte un système hétérocyclique azoté mono ou bicyclique saturé comprenant de 5 à 12 atomes - non compris les atomes d'hydrogène - intégrant ou non dans leur squelette carboné un, deux ou trois hétéroatomes choisis parmi azote, oxygène ou soufre,
leurs isomères, épimères, diastéréoisomères,
ainsi que leurs sels d'addition à un acide pharmaceutiquement acceptable et lorsque $R_1$ et $R_2$ représentent chacun un atome d'hydrogène, leurs sels d'addition à une base pharmaceutiquement acceptable.

**9/** Procédé de préparation selon la revendication 1 du composé qui est la 3-(2-pipéridinoéthyl) [3H] oxazolo [4,5-b] pyridine-2-one ainsi que ses sels d'addition à un acide pharmaceutiquement acceptable.

**10/** Procédé de préparation selon la revendication 1 du composé qui est la 3-[2-(4-méthyl pipérazin-1-yl)éthyl] [3H] oxazolo [4,5-b] pyridine-2-one ainsi que ses sels d'addition à un acide pharmaceutiquement acceptable.

**11/** Procédé de préparation selon la revendication 1 du composé qui est la 3-[2-(1-pyrrolidinyl)éthyl] [3H] oxazolo [4,5-b] pyridine-2-one ainsi que ses sels d'addition à un acide pharmaceutiquement acceptable.

**12/** Procédé de préparation selon la revendication 1 du composé qui est la 3-(2-morpholinoéthyl) [3H] oxazolo [4,5-b] pyridine-2-one ainsi que ses sels d'addition à un acide pharmaceutiquement acceptable.

**13/** Procédé de préparation selon la des revendication 1 du composé qui est le 2-(2- pipéridinoéthylamino) pyridin-3-ol ainsi que ses sels d'addition à un acide ou une base pharmaceutiquement acceptable.

**14/** Procédé de préparation de composition pharmaceutique contenant comme principe actif au moins un composé préparé selon l'une des revendications 1 à 13 en combinaison avec un ou plusieurs excipients ou véhicules inertes, non-toxiques, pharmaceutiquement acceptables.

**15/** Procédé de préparation de composition pharmaceutique selon la revendication 14 utilisables dans le traitement d'algies et des troubles de l'insuffisance circulatoire cérébrale.

Office européen des brevets

# RAPPORT DE RECHERCHE EUROPEENNE

Numero de la demande

EP 91 40 1741

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | HELVETICA CHIMICA ACTA, vol. 59, no. 5, 1976, pages 1593-1612, Verlag Helv. Chim. Acta, Basel, CH; K. RÜFENACHT et al.: "Arbeiten über Phosphorsäure- und Thiophosphorsäureester mit einem heterocyclischen Substituenten 10. Aza-Analogie II: Derivate von Oxazolo[4,5-b]pyridin-2(3H)-on, einem Aza-analogen von Benzoxazol-2(3H)-on" * Page 1603, composé 31 * | 1 | C 07 D 498/04<br>C 07 D 213/76<br>A 61 K 31/44 //<br>(C 07 D 498/04<br>C 07 D 263:00<br>C 07 D 221:00 ) |
| A | GB-A-1 260 352 (ROBAPHARM) * Revendication 1; page 1, colonne de droite, lignes 50-63 * | 1,15,16 | |
| A | EP-A-0 232 740 (FUJISAWA) * Revendications 1,8,9 * | 1,15,16 | |
| P,X | EP-A-0 412 899 (SCIENCE ET ORGANISATION) * Revendications 1,2,18,19 * | 1,15,16 | |

DOMAINES TECHNIQUES RECHERCHES (Int. Cl.5)

C 07 D 498/00
C 07 D 213/00
A 61 K 31/00

Le présent rapport a été établi pour toutes les revendications

| Lieu de la recherche | Date d'achèvement de la recherche | Examinateur |
|---|---|---|
| LA HAYE | 26-09-1991 | VOYIAZOGLOU D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)